# EUROPEAN PATENT SPECIFICATION

(11) **EP 0 675 954 B1**
(45) Date of publication and mention of the grant of the patent: **13.03.2002**
(21) Application number: 94905362.3
(22) Date of filing: 09.12.1993
(51) Int. Cl.: C12N 15/22, C12N 15/70, C12N 15/67, C12P 13/22, C12N 1/21

(54) **DEBLOCKING THE COMMON PATHWAY OF AROMATIC AMINO ACID SYNTHESIS**
ENTSPERRUNG DES GEMEINSAMEN SYNTHESEWEGES VON AROMATISCHEN AMINOSÄUREN
DEBLOCAGE DE LA VOIE COMMUNE DE LA SYNTHESE D'AMINOACIDE AROMATIQUE

(30) Priority: 21.12.1992 US 994194
(43) Date of publication of application: 11.10.1995
(73) Proprietor: PURDUE RESEARCH FOUNDATION, West Lafayette, IN 47907-1650 (US); GENENCOR INTERNATIONAL, INC., Rochester, New York 14618 (US)
(72) Inventor: FROST, John, W., Lafayette, IN 47905 (US); DELL, Kristi, A., West Lafayette, IN 47907 (US); DRATHS, Karen, M., Lafayette, IN 47905 (US); BERRY, Alan, Hilton, NY 14468 (US)
(74) Representative: Bannerman, David Gardner
(86) International application number: US9312026
(87) International publication number: WO9414955

(56) References cited:
- US-A- 4 681 852
- US-A- 5 168 056
- DRATHS K.M. AND FROST J.W.: "Genomic direction of synthesis during plasmid-based biocatalysis" J. AM. CHEM. SOC., vol. 112, 19 December 1990, pages 9630-9632, XP002051300
- DRATHS K.M. AND FROST J.W.: "Conversion of D-glucose into cathechol: the not-so-common pathway of aromatic biosynthesis" J. AM. CHEM. SOC., vol. 113, 20 November 1991, pages 9361-9363, XP002051301
- DELL K.A. AND FROST J.W.: "Identification and removal of impediments to biocatalytic synthesis of aromatics from D-glucose: rate limiting enzymes in the common pathway of aromatic amino acid biosynthesis" J. AM. CHEM. SOC., vol. 115, 1 December 1993, pages 11581-11589, XP002051291

## Description

### Field of the Invention

This invention relates to the enhancement of the efficiency of biosynthetic reactions. More particularly this invention is directed to a method for enhancing the biosynthesis of aromatic compounds in the common pathway in a host cell by identifying rate-limiting reaction steps in that pathway and genetically engineering the host cell to effectively deblock those rate-limiting steps.

### Background and Summary of the Invention

The common pathway of aromatic amino acid biosynthesis, otherwise known as the shikimate pathway, produces the aromatic amino acids, phenylalanine, tyrosine, and tryptophan in bacteria and plants. The route to the aromatic amino acids consists of a common pathway that ends in the branch point molecule chorismate which is subsequently converted to phenylalanine, tyrosine and tryptophan by three separate terminal pathways. The aromatic amino acids are essential supplements to the diets of humans and animals who lack the ability to synthesize the compounds. They are also precursors for many interesting and commercially important molecules such as aspartame, a synthetic sweetener, indigo, a common dye, and L-DOPA, a drug used to combat the effects of Parkinson's disease, to name a few.

The success of any biocatalytic route to overproduce the aromatic amino acids or their derivatives from a readily available carbon source such as glucose or other sugars depends on the ability to direct a surge of carbon through the pathway of the host organism. Metabolic blocks encountered in the pathway can effect the subsequent yield and purity of products produced by the biocatalytic conversion.

Earlier approaches for increasing efficiency of production of the common pathway of aromatic biosynthesis have been described in U.S. Patent No. 5,186,056, issuing December 1, 1992, on U.S. Application Serial No. 07/652,933, filed February 8, 1991, the disclosure of which is expressly incorporated herein by reference. That patent describes a related invention directed to increasing the carbon flow into the pathway by increasing the in vivo catalytic activity of DAHP synthase and transketolase. While the aforementioned patent specification indicates that other enzymes that catalyze steps in the common pathway can be overexpressed in combination with the principally targeted transketolase and DAHP synthase, it has been found that increased carbon flow directed into the common pathway is lost if there are one or more pathway enzymes that are not able to catalyze conversion of intermediate substrates to products at rates comparable to the rate at which those substrate intermediates are produced. Thus, there are certain rate-limiting steps in the biosynthetic pathway that work to impede the progress of the reaction steps through the pathway. The present invention removes those impediments.

The analysis of culture supernatants of the *Escherichia coli* strain D2704 (*pheA-, tyrA-, ΔtrpE-C*) using nuclear magnetic resonance spectroscopy (NMR) has identified 3-dehydroquinate synthase, shikimate kinase, 5-enolpyruvoylshikimate-3-phosphate synthase, and chorismate synthase as rate-limiting enzymes in the common pathway of aromatic amino acid biosynthesis. Insertion of a plasmid containing the genetic fragments coding for *aroL* (shikimate kinase), aroA (EPSP synthase), and *aroC* (chorismate synthase) along with the plasmid pKD136 (a plasmid that has been shown to commit an increased amount of carbon to the common pathway of aromatic amino acid biosynthesis) into the *Escherichia coli* strain D2704 resulted in the removal of the majority of the substrates of the rate-limiting enzymes from the culture broth as well as a significant increase in end product production.

### Brief Description of the Drawings

Fig. 1 illustrates the common pathway of aromatic amino acid biosynthesis.
Fig. 2 presents plasmid maps of pKD130A and pKD136.
Figs. 3A and 3B are bar graphs depicting the concentration of common pathway intermediates of D2704 strains of *E. coli* and the average phenylalanine and phenyllactic acid concentrations for those strains.
Fig. 4 illustrates the construction of plasmid pKD28 from plasmids pIA321 and pSU18.
Fig. 5 is similar to Fig. 4 showing construction of plasmid pKAD31.
Figs. 6A, 6B, and 6C illustrate the preparation of aroEaroL plasmid pKAD34.
Figs. 7-13 are similar to Figs. 4-7 and show the construction of plasmids pKAD38, pKAD43, pKAD39, pKAD50, pKAD44, pKAD51, and pKAD42, respectively.
Fig. 14 is a graph illustrating the total accumulation of phenylalanine, phenyllactic acid and prephenic acid in culture medium of *E. coli* transformants of this invention.

### Detailed Description of the Invention

In accordance with this invention there is provided a method for enhancing the biosynthesis of aromatic compounds in a host cell via the common pathway. In that pathway a metabolizable carbon source is converted to intermediate aromatic compounds in a multiple step reaction sequence characterized by enzyme species acting on intermediate substrates. One embodiment of the present method comprises developing a method to identify rate-limiting steps in the pathway, the method comprising the steps of analyzing supernatants of a culture of a host cell to identify accumulated common pathway intermediates - the substrates for the enzyme-mediated rate-limiting steps in the pathway. Having identified the rate-limiting steps, the host cell is transformed with recombinant DNA comprising DNA encoding for enzyme species acting on the identified accumulated intermediate substrates in the rate-limiting reaction steps of the pathway to increase expression of the enzyme species in the host cell. Additionally, enhanced expression of the enzyme species involved in the rate-limiting steps can also be achieved by genetically engineering the host cell to overexpress endogenous genes for such enzyme species, either by modification of endogenous control sequences or by affecting derepression of existing expression control sequences utilizing art accepted methods.

Regardless of the exact mechanism utilized for enhancing expression of the rate-limiting enzyme species, it is contemplated that such will typically be effected or mediated by the transfer of recombinant genetic elements into the host cell. Genetic elements as herein defined include nucleic acids (generally DNA or RNA) having expressible coding sequences for products such as proteins, specifically enzymes, apoproteins or antisense RNA, which express or regulate expression of rate-limiting enzymes in the common pathway. The expressed proteins can function as enzymes, repress or derepress enzyme activity, or control expression of enzymes. Recombinant DNA encoding these expressible sequences can be either chromosomal (integrated into the host cell chromosome by, for example, homologous recombination) or extrachromosomal (for example, carried by plasmids, cosmids, and other vectors capable of effecting the targeted transformation). It is understood that the recombinant DNA utilized for transforming the host cell in accordance with this invention can include, in addition to structural genes, expression control sequences including promoters, repressors, and enhancers that act to control expression or derepression of coding sequences for proteins, apoproteins or antisense RNA. For example, such control sequences can be inserted into wild type host cells to promote overexpression of selected enzymes already encoded in the host cell genome, or alternatively, they can be used to control synthesis of extrachromosomally encoded enzymes.

The recombinant DNA can be introduced into the host cell by plasmids, cosmids, phages, yeast artificial chromosomes or other vectors that mediate transfer of genetic elements into a host cell. These vectors can include an origin of replication along with cis-acting control elements that control replication of the vector and the genetic elements carried by the vector. Selectable markers can be present on the vector to aid in the identification of host cells into which genetic elements have been introduced. Exemplary of such selectable markers are genes that confer resistance to particular antibiotics such as tetracycline, ampicillin, chloramphenicol, kanamycin, or neomycin.

A preferred means for introducing genetic elements into a host cell utilizes an extrachromosomal multi-copy plasmid vector into which genetic elements in accordance with the present invention have been inserted. Plasmid borne introduction of the genetic element into host cells involves an initial cleaving of a plasmid vector with a restriction enzyme, followed by ligation of the plasmid and genetic elements encoding for the targeted enzyme species in accordance with the invention. Upon recircularization of the ligated recombinant plasmid, infection (e.g., packaging in phage lambda) or other mechanism for plasmid transfer (eg. electroporation, microinjection, etc.) is utilized to transfer the plasmid into the host cell. Plasmids suitable for insertion of genetic elements into the host cell include but are not limited to pBR322 and its derivatives such as pAT153, pXf3, pBR325, and pBR327, pUC vectors, pACYC and its derivatives, pSC101 and its derivatives, and ColE1.

Suitable host cells for use in the present invention are members of those genera capable of being utilized for industrial biosynthetic production of desired aromatic compounds. Accordingly, host cells include prokaryotes belonging to the genera Escherichia, Corynebacterium, Brevibacterium, Arthrobacter, Bacillus, Pseudomonas, Streptomyces, Staphylococcus, or Serratia. Eukaryotic host cells can also be utilized, with yeasts of the genus Saccharomyces or Schizosaccharomyces being preferred.

More specifically, prokaryotic host cells are derived from, but not limited to, species that include Escherichia coli, Corynebacterium glutamicum, Corynebacterium herculis, Brevibacterium divaricatum, Brevibacterium lactofermentum, Brevibacterium flavum, Bacillus brevis, Bacillus cereus, Bacillus circulans, Bacillus coagulans, Bacillus lichenformis, Bacillus megaterium, Bacillus mesentericus, Bacillus pumilis, Bacillus subtilis, Pseudomonas aeruginosa, Pseudomonas angulata, Pseudomonas fluorescens, Pseudomonas tabaci, Streptomyces aureofaciens, Streptomyces avermitilis, Streptomyces coelicolor Streptomyces griseus, Streptomyces kasugensis, Streptomyces lavendulae, Streptomyces lipmanii, Streptomyces lividans, Staphylococcus epidermis, Staphylococcus saprophyticus, or Serratia marcescens. Preferred eukaryotic host cells include Saccharomyces cerevisiae or Saccharomyces carlsbergensis.

For industrial production of primary metabolites derived from chorismate (such as aromatic amino acids), deregulated mutant strains of the above recited species that lack feedback inhibition of one or more enzymes in the metabolic biosynthetic pathway are preferred. Such strains can be created by random or directed mutagenesis, or are commercially available. Examples of E. coli strains having DAHP synthase, prephenate dehydratase, or chorismate mutase feedback inhibition removed are described in U.S. Patent 4,681,852 to Tribe and U.S. Patent 4,753,883 to Backman et al., the disclosures of which are incorporated herein by reference.

In preferred embodiments of the present invention, the enhanced expression of the rate-limiting enzyme species in the host cell is achieved by transformation of the host cell with a plasmid vector comprising DNA encoding for the enzyme species 3-dehydroquinate synthase, shikimate kinase, and 5-enolpyruvoylshikimate-3-phosphate synthase (EPSP synthase). More preferably, the transforming vector further comprises DNA encoding for chorismate synthase. In the most preferred embodiment of the method of the present invention an *E. coli* strain is transformed with recombinant DNA comprising DNA encoding for the enzymes transketolase (tkt), 3-deoxy-D-*arabino*-heptulosonate-7-phosphate synthase (DAHP synthase), 3-dehydroquinate synthase (DHQ synthase), shikimate kinase, 5-enolpyruvoylshikimate-3-phosphate synthase, and chorismate synthase to increase expression of those enzymes in the host cell.

Typically the recombinant DNA is introduced into the host cell as part of one or more recombinant plasmid vectors comprising the DNA encoding for the enzyme species.

Other embodiments of the present invention include cell transformants prepared in accordance with the method of this invention and a method utilizing such cell transformants to produce an aromatic compound biocatalytically from a carbon source. The method comprises the step of culturing a cell transformant of this invention capable of utilizing a carbon source in its common pathway in the presence of such a carbon source under conditions conducive to the use of the carbon source in the pathway. Other embodiments of the present invention include plasmid constructs comprising structural genes for two or more of the rate-limiting enzymes of the common aromatic biosynthetic pathway. For example, one preferred construction comprises structural genes for shikimate kinase and EPSP synthase, more preferably including as well the structural gene for chorismate synthase. A microorganism transformed with such plasmid constructs is still another contemplated embodiment of the present invention.

As mentioned above, there has been earlier efforts to enhance the biosynthetic production of compounds derived from the common pathway in a host cell by increasing the expression of proteins catalyzing reactions in that pathway. The present invention provides for significant improvement in the efficiency of production of aromatic compounds in host cells via the common pathway. While earlier reports have taught that carbon flow can be increased into the upper end (the initial reaction sequences) of the pathway by enhancing the concentrations of transketolase alone or in combination with other enzymes in the common pathway, for example, DAHP synthase, DHQ synthase and even shikimate kinase, there was no suggestion by that earlier work that rate-limiting enzyme species could be identified and that such identification could be used as a guide to transform the host cell for overexpression of the rate-limiting enzyme species to provide a significant increase in carbon flow through the aromatic pathway (as evidenced by the concentration of "in process" aromatic metabolites in the culture medium of transformant host cells). Thus the present invention can be viewed as well as an improvement on earlier efforts to increase the biosynthetic production of compounds derived from the common pathway, the improvement comprising the steps of (1) identifying the rate-limiting reaction steps in said pathway, and (2) increasing expression of those proteins catalyzing the identified rate-limiting steps in the pathway. Again, the increased expression is preferably achieved in accordance with this invention by transforming the host cell to express constitutively exogenous genes encoding for said protein catalyst (enzyme) to increase concentration of the proteins in the host cell. The improvement has been shown particularly where the host cell is a strain of *E. coli* transformed to express exogenous structural genes comprising the genes for transketolase, 3-deoxy-D-arabino-heptulosonate-7-phosphate synthase, 3-dehydroquinate synthase, shikimate kinase, 5-enolpyruvoylshikimate-3-phosphate synthase and chorismate synthase.

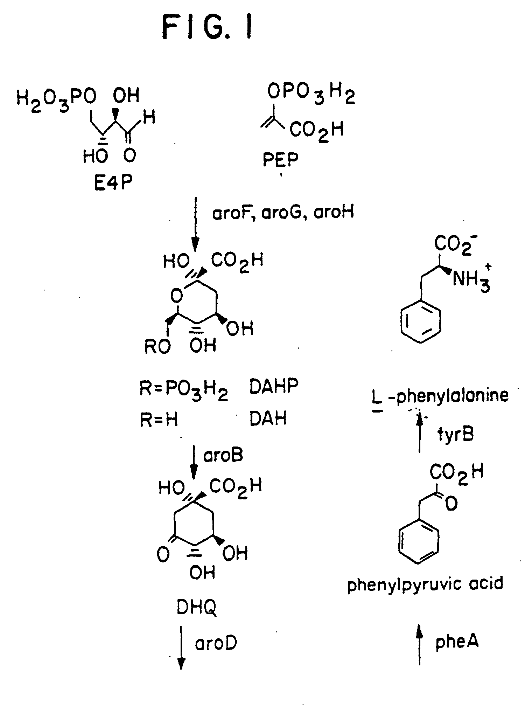

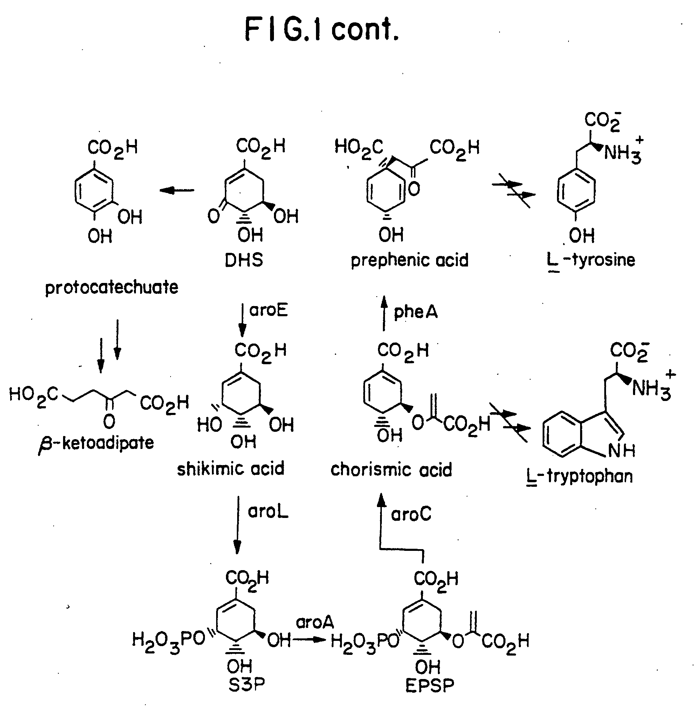

D2704, an *Escherichia coli* strain that is *pheA-, tyrA-* and *ΔtrpE-C* should theoretically be able to produce chorismic acid because the terminal pathways leading to phenylalanine, tyrosine, and tryptophan are respectively blocked (Fig. 1). Using this strain, deblocking of the common pathway of aromatic amino acid biosynthesis in *E. coli* when an increased surge of carbon was committed to the pathway was planned with the increased accumulation of chorismate as an indicator of successful blocking. Growth of D2704 cells in rich media followed by resuspension in minimal salts accumulation media gave little or no accumulation of chorismate but yielded significant levels of phenylalanine. The production of phenylalanine can be explained by the non-enzymatic Claisen rearrangement of chorismic acid to prephenic acid followed by dehydration to produce phenylpyruvic acid. Although the enzyme chorismate mutase accelerates the conversion of chorismate to prephenate by 2x10⁶ at 37°C, the reaction can occur in the absence of the enzyme. Prephenic acid has been reported to yield phenylpyruvate non-enzymatically under mildly acidic conditions such as those produced during normal culturing of cells. With the production of phenylpyruvic acid, the microbe should be able to synthesize phenylalanine using the intact amino transferase encoded by *tyrB*. However significant amounts of phenyllactate were observed in some of the culture supernatants.

The aromatic amino transferase encoded by tyrB transaminates the aromatic keto acid using glutamate as the nitrogen donor and pyridoxal phosphate as a coenzyme. [Mavrides, C. In *Methods in Enzymology*; Academic: San Diego, **1987**, 142, pp. 253-267.] The production of phenyllactic acid could be due to insufficient supplies of glutamate in the cell to completely transaminate all of the phenylpyruvic acid. Reduction of phenylpyruvic acid to phenyllactate might occur to regenerate a supply of NAD⁺ within the cell. An analogous reduction of pyruvate to lactic acid catalyzed by the enzyme lactate dehydrogenase [Holbrook, J.J.; Liljas, A.; Steindel, S.S.; Rossmann, M.G. In *The Enzymes;* Boyer, P.D., Ed.; Academic Press: New York, 1975; Vol. 11, Chap. 4] is known to occur under anaerobic conditions to regenerate a supply of NAD⁺ for the continued functioning of glycolysis.

The activity of the aromatic amino transferase could also be limited by the presence of the *pheA* mutation in D2704. It has been shown that the bifunctional enzyme chorismate mutase-prephenate dehydratase encoded by *pheA* interacts with the aromatic amino transferase in the presence of phenylpyruvate to form a complex in *E. coli* [Powell, J.T.; Morrison, J.F.; *Biochem. Biophys. Acta,* **1979**, 568, 467-474]. Since D2704 is *pheA*, it should be unable to produce the chorismate mutase-prephenate dehydratase enzyme necessary for complex formation. Although the role of the enzyme-enzyme interaction has not been determined, the possibility exists that the inability to form the complex could affect aminotransferase activity resulting in the buildup of phenylpyruvic acid within the cell. Although the above theories are plausible, the reason for phenyllactate accumulation has yet to be determined experimentally. However it is safe to assume that phenyllactate accumulation represents deblocked glucose equivalents from the common pathway. Therefore the successful removal of metabolic blocks from the common pathway of aromatic amino acid biosynthesis was measured by the combined total accumulation of phenylalanine and phenyllactic acid in the following study. Accumulation of common pathway intermediates in the culture supernatant was used to identify enzymes that were rate-limiting steps in the flow of carbon down the common pathway using the notion that the accumulated intermediate was the substrate of a rate-limiting enzyme.

Five milliliter starter cultures of each strain were grown in LB media containing the appropriate drugs for ten hours. The starter cultures were used to inoculate one liter cultures of LB in four liter erlenmeyer flasks with isopropyl *B*-D-thiogalactopyranoside (IPTG) (0.2 mM), chloramphenicol (20 mg/L), and ampicillin (50 mg/L) added where needed. The one liter cultures were grown for 12 hours at 37°C with agitation (250 RPM). Cells were harvested (3,000 g; 5 minutes; 4°C) and washed three times with M9 salts [M9 salts contain (per liter): 6g Na₂HPO₄, 3g KH₂PO₄, 0.5g NaCl, 1g NH₄Cl] (300 mls wash for each sample). Cell pellets were resuspended in one liter of M9 accumulation media in a four liter erlenmeyer flask containing glucose (10 g), MgSO₄ (1 mM), and thiamine (30 mg) with the addition of chloramphenicol, ampicillin and IPTG where needed. Cells were incubated for an additional 48 hours in the accumulation media at 37°C with agitation (250 RPM). Aliquots (25 ml) were removed at 24 and 48 hour intervals and centrifuged (6,000g; 5 min; 4°C). Ten milliliters of isolated supernatant was collected from each sample and the water was removed in vacuo. Samples were exchanged two times with D₂O and analyzed by ¹H NMR. The sodium salt of 3-(trimethylsilyl) propionic-2,2,3,3-d₄ acid was used as the internal standard to quantify intermediates and end products produced in the accumulation. All cultures were grown in triplicate so that mean values of accumulated molecules as well as their standard deviations could be obtained.

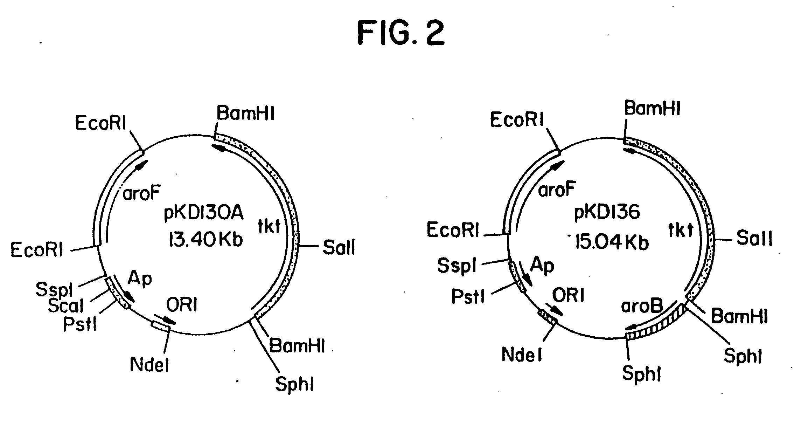

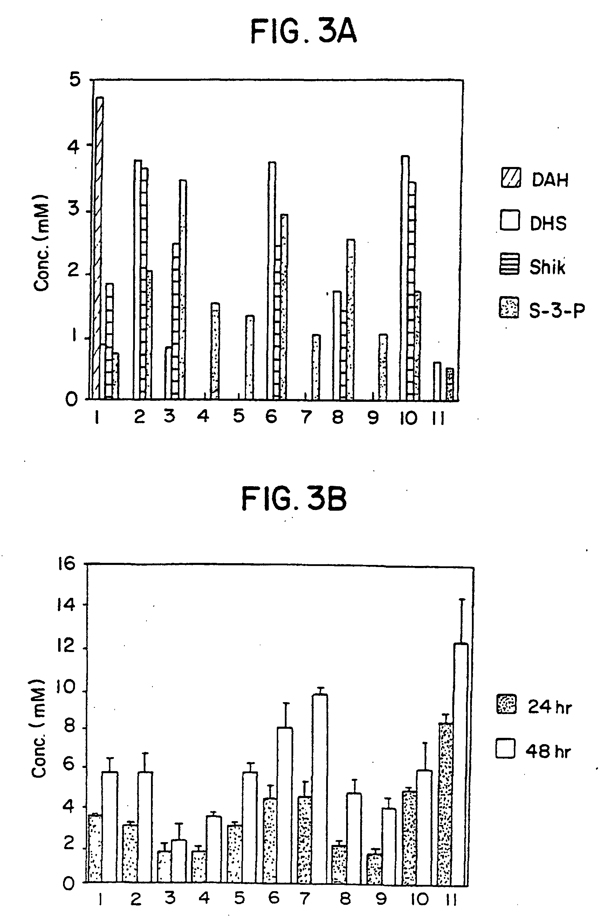

To create a surge of carbon through the common pathway of aromatic amino acid biosynthesis, a plasmid containing transketolase, tkt, and the tyrosine sensitive isozyme of 3-deoxy-D*-arabino*-heptulosonate-7-phosphate synthase (DAHP synthase), *aroF*, was employed. Transketolase has been shown to increase the levels of erythrose 4-phosphate available to the cell, for use in producing aromatic amino acids while DAHP synthase is the first irreversible step of the pathway. The *tkt, aroF* plasmid pKD130A (Fig. 2), a pBR325 derivative with the ampicillin resistance gene intact and a pMB1 origin of replication, accumulated the common pathway intermediates 3-deoxy-D-*arabino*-heptulosonate-7-phosphate (DAHP), 3-dehydroshikimate (DHS), shikimate and shikimate-3-phosphate with a total phenylalanine and phenyllactate accumulation of 5.6 ± 0.7 mM upon introduction into D2704 (Fig. 3). After incubation for 48 hours, ¹H NMR resonances for DAHP are found at δ 1.79 (dd, 13, 13 Hz, 1 H), δ 2.20 (dd, 13, 5 Hz, 1 H), δ 3.46 (dd, 9, 9 Hz, 1 H) and δ 3.83 (m, 2 H). The presence of shikimate in the culture media is shown by resonances at δ 4.41 (dd, 4, 4 Hz, 1 H) and δ 6.47 (m, 1 H). A resonance for shikimate - phosphate lies at δ 6.47 (m, 1 H). Resonances for phenylalanine are found at δ 3.14 (dd, 14, 8 Hz, 1 H), δ 3.29 (dd, 14, 5 Hz, 1 H) and δ 7.30 - 7.49 (m, 5 H). Observable resonances for phenyllactic acid are found at δ 4.27 (dd, 8, 4 Hz, 1 H) and δ 7.30 - 7.49 (m, 5 H). DHS disappeared from the accumulation media between 24 and 48 hours.

The accumulation of DAHP, DHS, shikimate, and shikimate-3-phosphate in the culture supernatant lead to the assignment of 3-dehydroquinate synthase (DHQ synthase), shikimate dehydrogenase, shikimate kinase, and 5-enolpyruvoylshikimate-3-phosphate synthase (EPSP synthase) respectively as rate-limiting enzymes. Although DHQ synthase and shikimate dehydrogenase had been previously identified to be rate-limiting steps in the common pathway, [Draths, K.M.; Frost, J.W.; *J. Am. Chem. Soc*. **1990**, 112, 9360-9632; Draths, K.M., Ph.D. Dissertation, Stanford University, June 1991] the identification of shikimate kinase and EPSP synthase as rate-limiting steps has not been reported in the literature.

To remove the accumulation of DAHP in the culture supernatant, a *tkt, aroF, aroB* plasmid pKD136 (Fig. 2) was introduced into D2704. Using pKD136, DAHP was successfully removed from the culture supernatant resulting in an increased accumulation of DHS, shikimate, and shikimate-3-phosphate but no increase in phenylalanine and phenyllactate (Fig. 3). Fig. 3A shows concentrations of common pathway intermediates accumulated in D2704 strains after 24 hours of growth in minimal media. Fig. 3B illustrates total accumulation of phenylalanine and phenyllactate after 24 and 48 hours of growth in minimal media. Strains studied include 1) D2704/pKD130A; 2) D2704/pKD136; 3) D2704/pK136/pKD28; 4) D2704/pKD136/pKAD34; 5) D2704/pKD136/pKAD31 6) D2704/pKD136/pKAD38; 7) D2704/pKD136/pKAD43; 8) D2704/pKD136/pKAD39; 9) D2704/pKD136/pKAD51; 10) D2704/pKD136/pKAD44; 11) D2704/pKD136/pKAD50.
Thus even though a rate-determining step had been removed from the pathway, no increased accumulation of end product was observed.

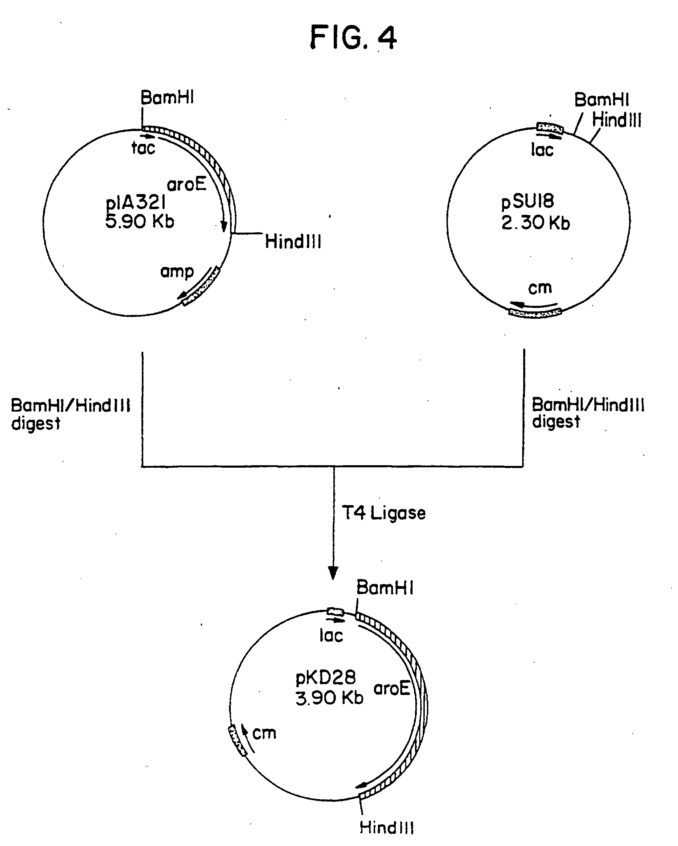

The lack of convenient unique restriction sites for the insertion of *aroE* into pKD136 resulted in the use of a two plasmid system for the rest of the deblocking experiments. The system consisted of pKD136 and the pSU2718/pSU2719 [Martinez, E.; Bartolome, B.; de la Cruz, F. Gene, **1988**, 68, 159-162] derived plasmids pSU18 and pSU19, possessing chloramphenicol resistance, a lac promoter, and a p15A origin of replication, into which the remaining deblocking genes were inserted. A pSU18 based *aroE* plasmid, pKD28, [Draths, K.M., Ph.D. Dissertation, Stanford University, June 1991] was created by isolation of a 1.6 kb fragment containing a tac promoter and the *aroE* gene from pIA321 [Anton, I.A.; Coggins, J.R. *Biochem. J.,* **1988**, 249, 319-326] followed by ligation into pSU18 as shown in Fig. 4. D2704/pKD136/pKD28 while reducing the level of DHS accumulation did not completely remove the intermediate from the culture supernatant. Shikimate and shikimate-3-phosphate were still present in the culture broth. The total production of phenylalanine and phenyllactate was reduced to 2.1 ± 0.9 mM after 48 hours of growth (Fig. 3) implying that the increased carbon flow from deblocking at *aroE* did not result in the additional accumulation of end products.

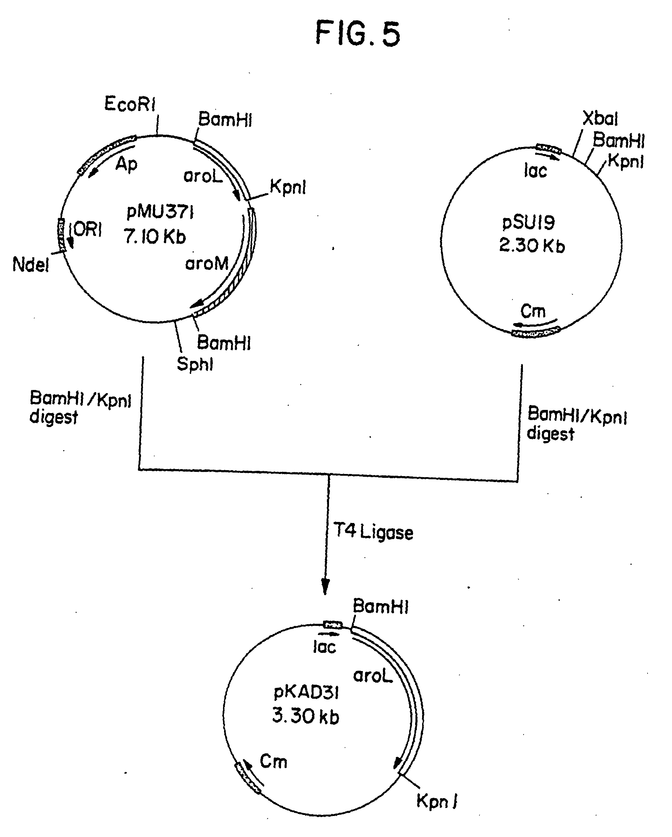

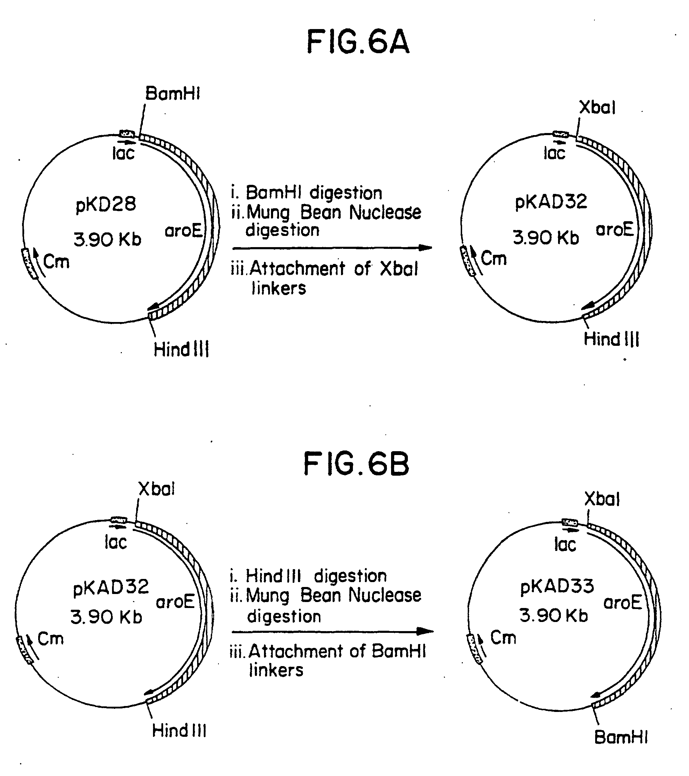

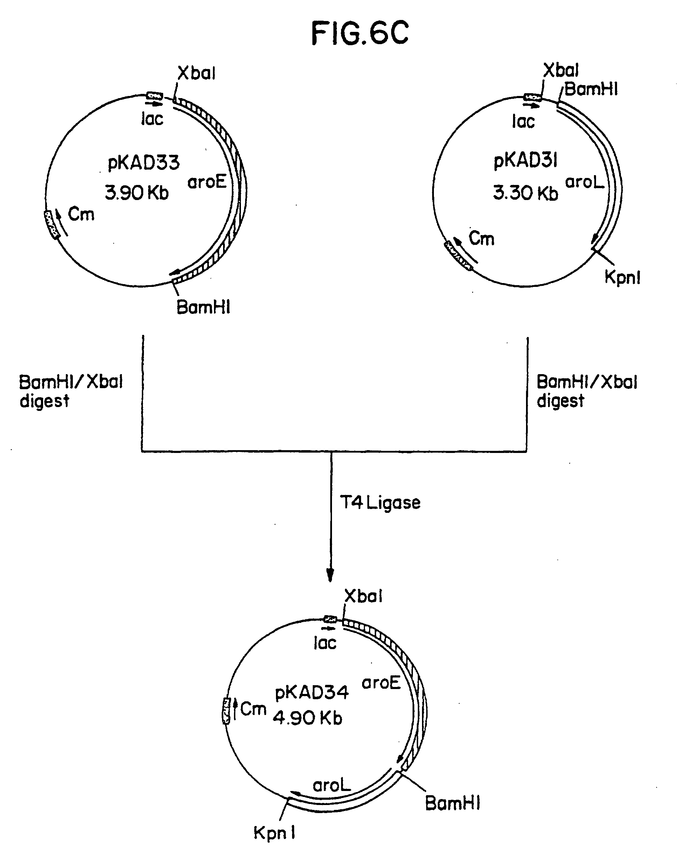

To remove the rate-limiting characteristics of shikimate kinase, both *aroL* and *aroEaroL* plasmids were constructed. *aroL* is located in a transcriptional unit with *aroM*, a gene whose function is unknown [DeFeyter, R.C.; Pittard, *J. J. Bacteriol*., **1986**, 165, 226-232]. A 2.7 kb fragment containing the transcriptional unit had previously been isolated and cloned into pBR322 to form the plasmid pMU371 [DeFeyter, R.C.; Pittard, J. *J. Bacterial*., **1986**, 165, 226-232]. A one kb fragment containing *aroL* was isolated from the plasmid pMU371 and inserted into the vector pSU19 creating the 3.3 kb *aroL* plasmid pKAD31 (Fig. 5). The 4.9 kb *aroEaroL* plasmid pKAD34 was obtained by manipulation of the flanking restriction sites of the *aroE* gene from pKD28 followed by its isolation and ligation into the unique XbaI and BamHI sites of pKAD31 (Fig. 6).

The *aroEaroL* construct D2704/pKD136/pKAD34 was able to completely remove DHS and shikimate from the culture supernatant leaving the only accumulated common pathway intermediate to be shikimate-3-phosphate. The total production of phenylalanine and phenyllactate was 3.4 ± 0.2 mM, a slight increase from the end product production of D2704/pKD136/pKD28 but still significantly smaller than the phenylalanine and phenyllactate concentrations observed with both D2704/pKD130A and D2704/pKD136 (Fig. 3).

The aroL construct D2704/pKD136/pKAD31 was also able to completely remove DHS and shikimate from the culture broth thereby relieving the rate-limiting characteristics of both shikimate dehydrogenase and shikimate kinase with only one overproduced gene. The rate-limiting character of shikimate dehydrogenase therefore appears to be an artifact of shikimate accumulation. The importance of the removal of shikimate from the culture media on the rate-limiting characteristics of shikimate dehydrogenase suggests that shikimate may have some inhibitory effects on the enzyme. The accumulation of shikimate-3-phosphate was still observed and the total production of phenylalanine and phenyllactate was found to be 5.6 ± 0.5 mM, the level of end product production initially observed with D2704/pKD130A and D2704/pKD136 (Fig. 3). Thus upon removing the metabolic blocks of DHQ synthase, shikimate dehydrogenase, and shikimate kinase, the total accumulation of pathway end products did not significantly increase leaving the deblocked glucose equivalents unaccounted for.

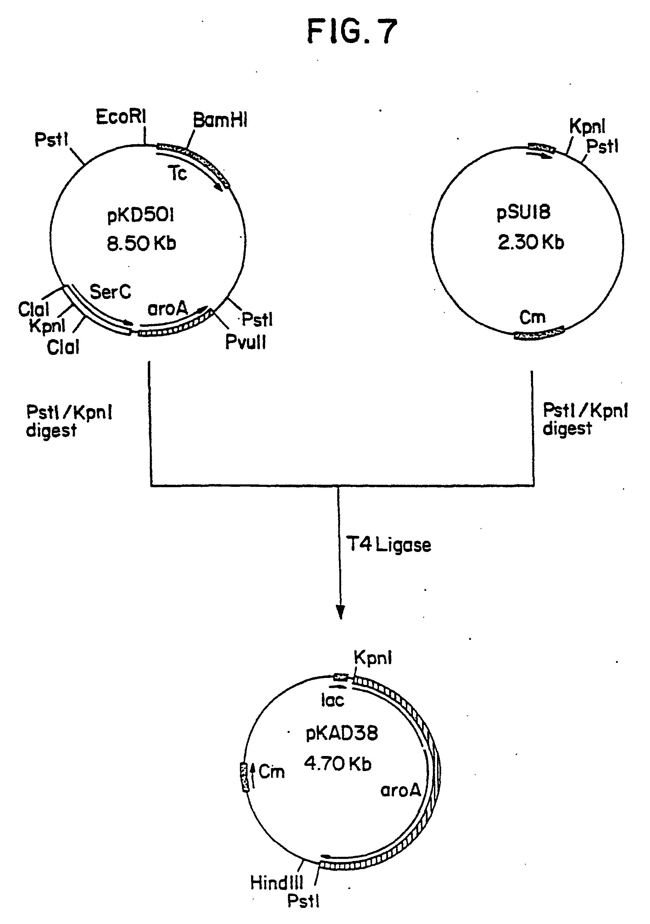

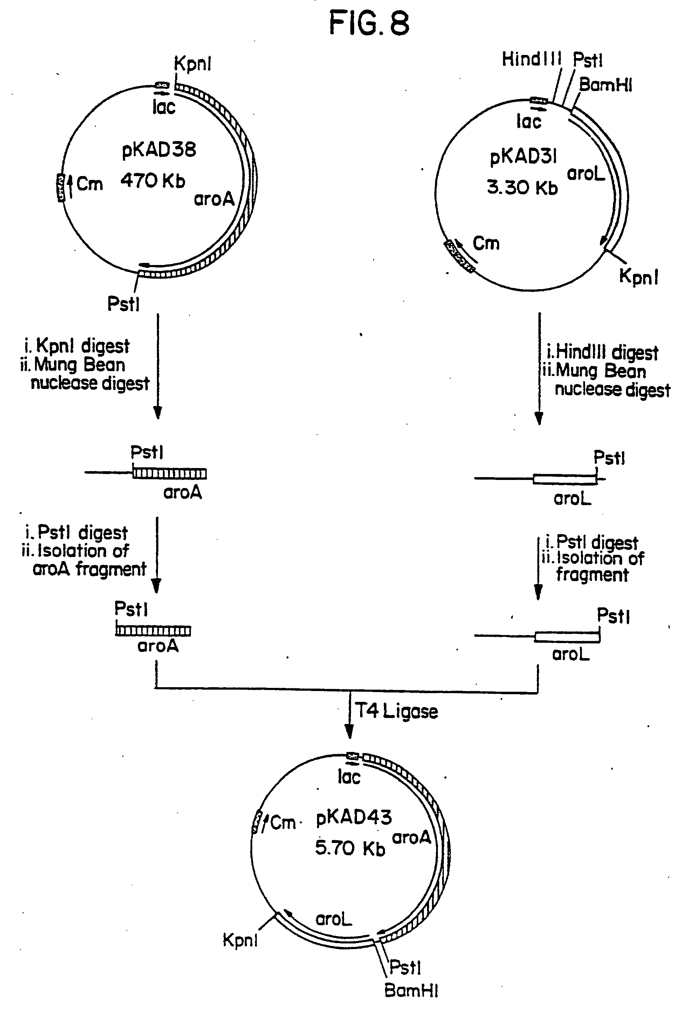

EPSP has been reported [Duncan, K.; Lewendon, A.; Coggins, J.R. *FEBS Lett*., 1984, 165, 121-127] to be an inhibitor of the forward reaction of EPSP synthase suggesting a possible explanation for the observance of rate-limiting characteristics of the enzyme. To remove the shikimate-3-phosphate from the culture supernatant, both aroA and *aroAaroL* plasmids were constructed. The aroA gene exists on an operon with *serC* which encodes 3-phosphoserine aminotransferase, a serine biosynthetic pathway enzyme. The 4.7 kb fragment encoding the *serCaroA* operon has been isolated and sequenced [Duncan, K.; Coggins, J.R. *Biochem. J*., **1986**, 234, 49-57; Duncan, K.; Lewendon, A.; Coggins, J.R. *FEBS* Lett., **1984**, 170, 59-63]. To create the 4.7 kb *aroA* plasmid pKAD38, a 2.4 kb *aroA* fragment was isolated from the plasmid pKD501 [Duncan, K.; Coggins, J.R. *Biochem. J*., **1986**, 234, 49-57] and ligated into the vector pSU18 directly behind the external lac promoter (Fig. 7). Removal of *aroA* from the transcriptional unit of *serCaroA* necessitates its placement behind an external promoter for expression. A rho-independent transcription terminator that is located between the *serC* and *aroA* genes and is believed to naturally attenuate *aroA* expression remains intact on the 2.4 kb *aroA* fragment since a convenient restriction site for its removal was not available. Placement of the truncated *aroA* gene with the transcription terminator behind an external lac promoter should still provide some level of overexpression of EPSP synthase. The 5.7 kb *aroAaroL* plasmid, pKAD43 (Fig. 8), was created by isolation of the 2.4 kb *aroA* gene with flanking PstI and blunt ended sites and ligation into a pKAD31 vector that had been manipulated to possess equivalent sites.

Evaluation of the strain D2704/pKD136/pKAD38 revealed a significant increase in total phenylalanine and phenyllactate production producing 7.9 ± 1.3 mM after 48 hours of accumulation (Fig. 3). Pathway intermediates accumulated in the supernatant were DHS, shikimate, and shikimate-3-phosphate. The strain D2704/pKD136/pKAD43 produced 9.7 ± 0.3 mM of phenylalanine and phenyllactate with the accumulation of only one common pathway intermediate, shikimate-3-phosphate. The *aroA* plasmids gave the first indication of successful conversion of deblocked glucose equivalents to end products. The inability of the *aroA* gene to completely remove shikimate-3-phosphate accumulation may result from the reversibility of the reaction catalyzed by EPSP synthase.

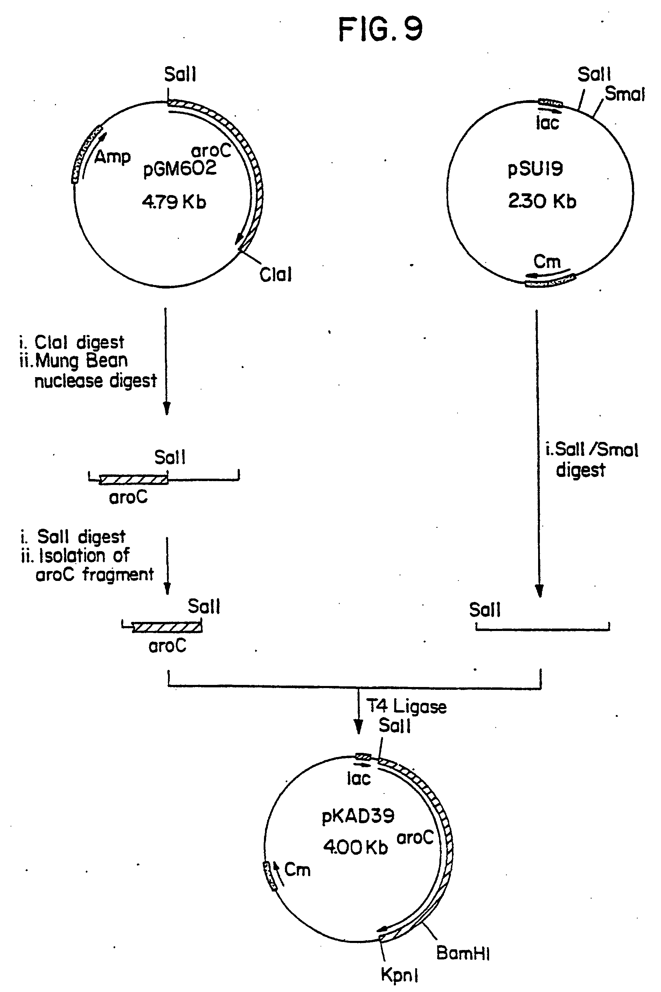

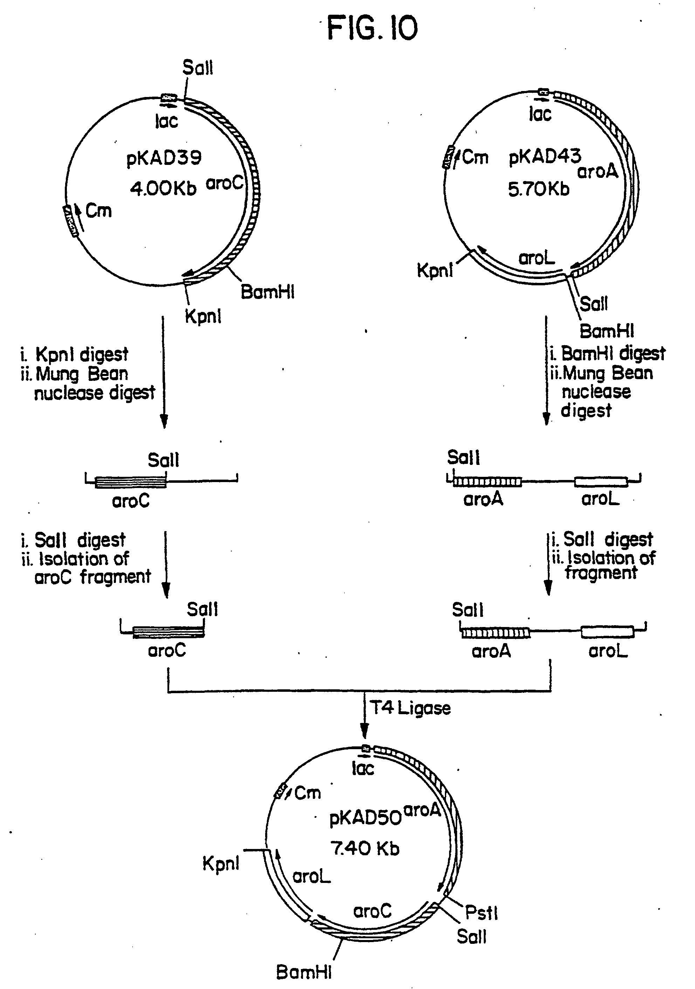

It has been suggested that chorismate synthase is both an irreversible and possibly rate-limiting enzyme [Pittard, A.J. In *Escherichia coli and Salmonella typhimurium*; Neidhardt, F.C., Inhgraham, J.L., Low, K.B., Magasanik, B., Schaechter, M., Umbarger, H.E., Eds.; American Society for Microbiology: Washington, DC 1987; Vol. 1, Chapter 24]. Rate-limiting characteristics of chorismate synthase might result in the continued presence of shikimate-3-phosphate if accumulations of EPSP are subsequently converted to shikimate-3-phosphate by EPSP synthase. In an attempt to completely remove shikimate-3-phosphate from the culture supernatant, an *aroAaroCaroL* plasmid was constructed. An *aroC* plasmid was first constructed by isolation of the *aroC* fragment flanked by SalI and blunt ended sites from pGM602 [White, P.J.; Millar, G.; Coggins, J.R.; Biochem. J., **1988**, 251, 313-322], a plasmid containing a 1.69 kb fragment encoding chorismate synthase. Ligation into the unique SalI and SmaI sites of pSU19 created the 4 kb plasmid pKAD39 (Fig. 9). To create the 7.4 kb *aroAaroCaroL* plasmid pKAD50, the 1.69 kb *aroC* fragment was isolated from pKAD39 as a SalI/blunt ended fragment and ligated into a pKAD43 vector that had been manipulated to contain equivalent ends (Fig. 10).

The strain D2704/pKD136/pKAD50 produced 12.3 ± 2.2 mM of phenylalanine and phenyllactate, a significant increase in end product production over D2704/pKD136/pKAD43 (Fig. 3). While D2704/pKD136/pKAD50 still accumulated some shikimate-3-phosphate, the total amount accumulated was less than D2704/pKD136/pKAD43. The NMR of the 48 hour D2704/pKD136/pKAD50 accumulation indicates the presence of phenylalanine by resonances at δ 3.29 (dd, 14, 5 Hz, 1 H), δ 4.0 (dd, 8, 5 Hz, 1 H), and δ 7.25 - 7.49 (m, 5 H). Resonances for phenyllactic acid are found at δ 2.88 (dd, 14, 8 Hz, 1 H), δ 4.27 (dd, 8, 4 Hz, 1 H), and δ 7.25-7.49 (m, 5 H). A small amount of DHS is also present in the culture broth as indicated by the presence of a resonance at δ 6.4 (d, 3 Hz, 1 H). The observed increased end product production upon the addition of *aroC* to the deblocking plasmid has lead to the assignment of chorismate synthase as a rate-limiting enzyme with the assumption that accumulation of EPSP might be converted to shikimate-3-phosphate.

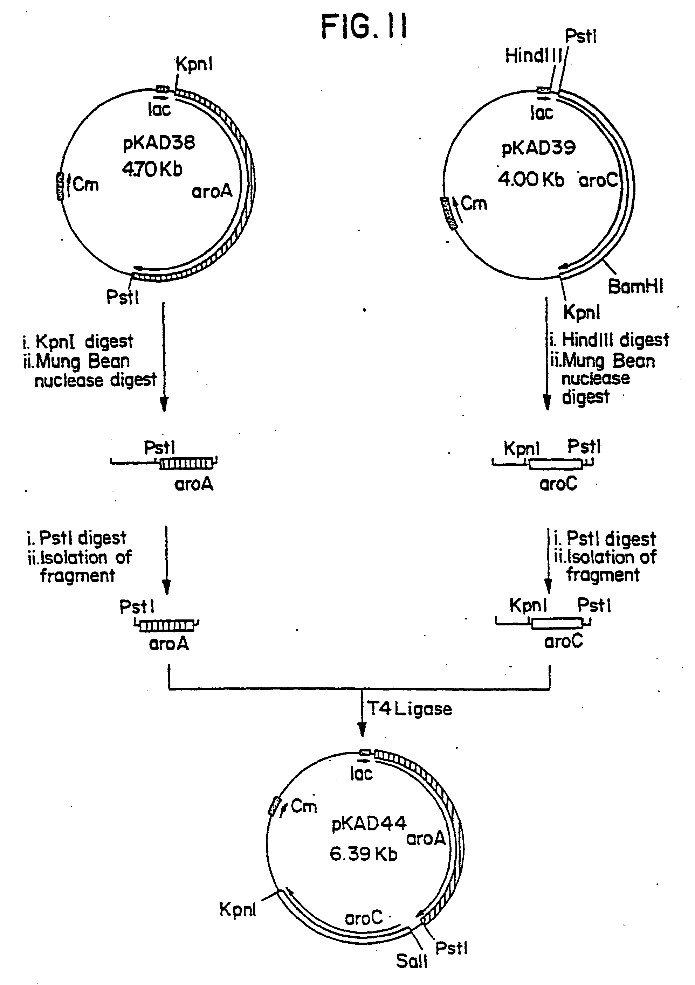

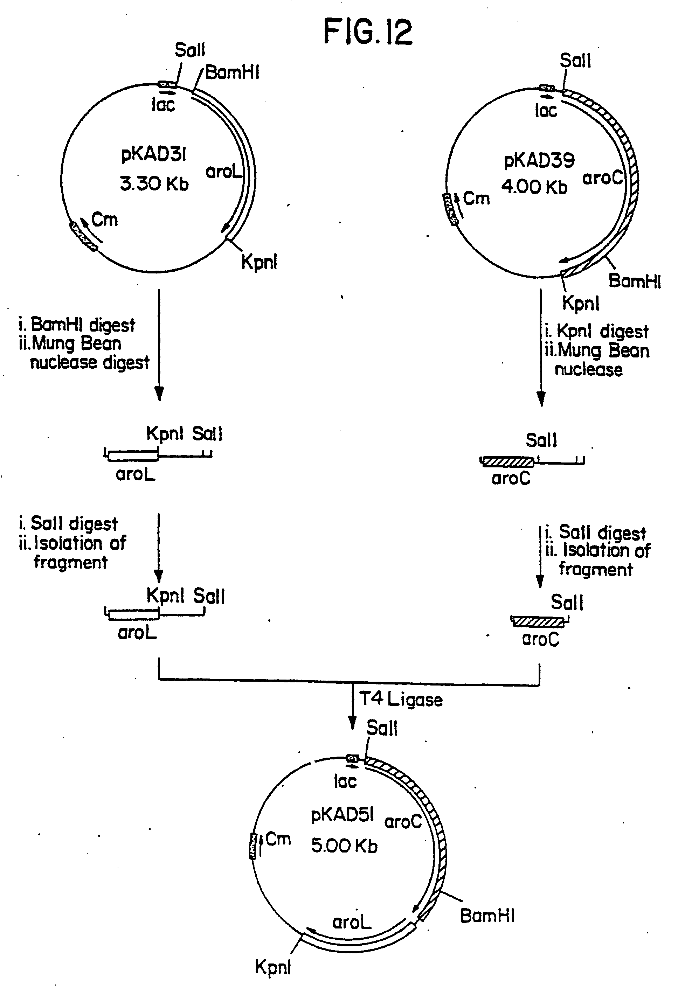

To further understand the role of chorismate synthase, plasmids containing aroC (pKAD39; Fig. 9), *aroAaroC*, and *aroCaroL* were constructed and evaluated in the strain D2704/pKD136. The 6.39 kb *aroCaroA* plasmid pKAD44 (Fig. 11) was created by the isolation of an aroA fragment with flanking PstI and blunt ended sites followed by ligation into a pKAD39 vector that had been manipulated to contain equivalent blunt-ended sites. The 5 kb *aroCaroL* plasmid pKAD51 (Fig. 12) was constructed by the isolation of *aroC* as a SalI blunt ended fragment which was ligated into a pKAD31 vector that had been manipulated to contain equivalent sites. As can be seen in Fig. 3, pKAD39, pKAD44, and pKAD51 did not achieve the levels of end product accumulation that the *aroAaroCaroL* plasmid pKAD50 achieved upon insertion into D2704/pKD136. Therefore the strain D2704/pKD136/pKAD50 was determined to be the optimum strain for maximal end product production.

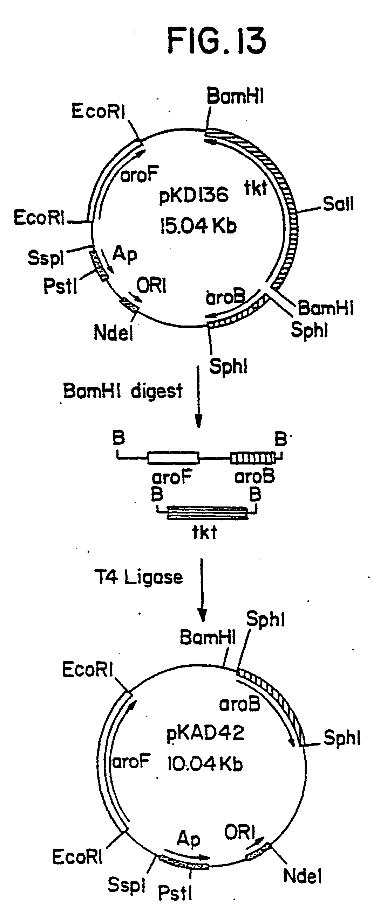

To determine the role of transketolase in the optimal strain D2704/pKD136/pKAD50, the gene was removed from the plasmid pKD136 by digestion with BamHI followed by religation creating the *aroFtkt* plasmid pKAD42 (Fig. 13). Culturing of the strain D2704/pKAD42/pKAD50 resulted in the accumulation of large amounts of acetate and lactate resulting in cell death. To alleviate this problem, the pH of the accumulation media was monitored during the 48 hour incubation and neutralized with 5N NaOH when needed. The maintenance of a neutral pH resulted in high accumulations of prephenic acid at both 24 and 48 hour time points of D2704/pKAD42/pKAD50 possibly due to the molecule's decreased ability to rearrange to phenylpyruvate at neutral pH. Thus to compare the amount of carbon flow successfully delivered to the end of the common pathway between the strains D2704/pKAD42/pKAD50 and D2704/pKD136/pKAD50, total amounts of phenylalanine, phenyllactic acid and prephenic acid were considered.

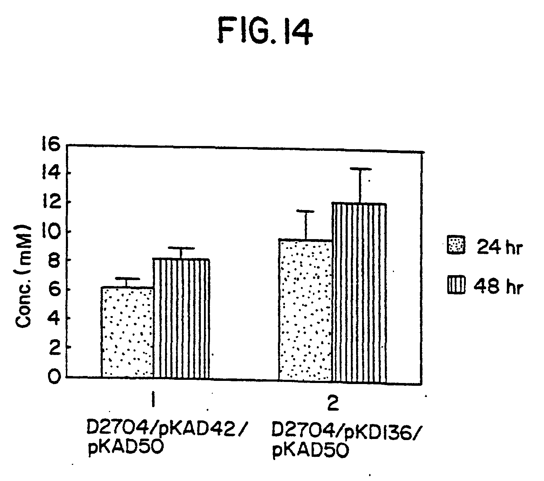

As shown in Fig. 14, the amount of end products produced by the strain D2704/pKAD136/pKAD50 was significantly larger than that produced by the strain D2704/pKAD42/pKAD50. This result shows that to successfully direct an increased surge of carbon to the aromatic amino acids and their derivatives, extra chromosomal copies of transketolase are required to increase the levels of carbon available to the common pathway as well as the genes encoding DAHP synthase, DHQ synthase, shikimate kinase, EPSP synthase, and chorismate synthase to successfully direct the surge to the desired end products.

Analysis of cell supernatants by NMR spectroscopy has revealed DHQ synthase, shikimate kinase, EPSP synthase, and chorismate synthase as metabolic blocks in the common pathway of aromatic amino acid biosynthesis. The previous identification of shikimate dehydrogenase as a metabolic block is thought to be an artifact of shikimate accumulation in the culture media. Both the yield and purity of the aromatic amino acids and their derivatives produced by biocatalytic processes can be increased by the employment of a two plasmid system in *E. coli*. The plasmid pKD136 or a functional equivalent is essential to committing an increased flow of carbon to the common pathway of aromatic amino acid biosynthesis while the plasmid pKAD50 or its functional equivalent is essential to successfully direct the surge of carbon to the end of the common pathway. The increased purity of the end products observed upon introduction of the deblocking genes *aroB, aroL, aroA* and *aroC* are readily discernible in the NMRs of D2704/pKD130A and D2704/pKD136/pKAD50.

## Claims

1. A method for enhancing production of an aromatic compound biocatalytically in an *E. coli* cell transformant via the common pathway of aromatic amino acid biosynthesis endogenous to said cell, said method comprising the step of
culturing the cell transformant in media containing a metabolizable carbon source under conditions conductive to the metabolization of said carbon source, said cell transformant comprising exogenous DNA sequences encoding common pathway enzyme species, said enzyme species consisting essentially of the enzymes, transketolase, 3-deoxy-D-arabino-heptulosonate-7-phosphatesynthase, 3-dehydroquinate synthase, shikamate kinase, 5-enolpyruvoylshikimate-3-phosphate synthase and chorismate synthase.

2. The method of claim 1 wherein one or more recombinant plasmid vectors comprise the exogenous DNA sequences encoding for said enzyme species.

3. An *E. coli* transformant **characterized by** the enhanced expression of structural genes encoding the enzyme species, transketolase, 3-deoxy-D-arabino-heptulosonate-7-phosphatesynthase, 3-dehydroquinate synthase, shikimate kinase, 5-enolpyruvoylshikimate-3-phosphate synthase and chorismate synthase.

4. A method for enhancing production of an aromatic compound biocatalytically from a carbon source, said method comprising the step of culturing the cell transformant of claim 3 in media containing a metabolizable carbon source under conditions conductive to the metabolization of said carbon source.

5. A plasmid construct comprising structural genes for common pathway enzyme species, said enzyme species consisting essentially of 3-dehydroquinate synthase, shikimate kinase, 5-enolpyruvoyl-shikamate-3-phosphate synthase and chorismate synthase.

6. A prokaryotic cell transformant selected from the group of prokaryotes belonging to the genera *Esherichia, Corynebacterium, Brevibacteria, Arthrobacter, Bacillus,. Pseudomonas, Streptomyces, Staphlococcus or Seratia,* and **characterized by** the expression of exogenous structural genes encoding the enzyme species transketolase, 3-deoxy-D-*arabino*-heptulosonate-7-phosphate synthase, 3-dehydroquinate synthase, shikimate kinase,5-enolpyruvoyl-shikimate-3-phosphate synthase and chorismate synthase.

7. Plasmid pKAD50, as shown in Figure 10.

8. The method of claim 1, wherein the exogenous DNA sequences for shikimate kinase, 5-enolpyruvoyl-shikimate-3-phosphate synthase and chorismate synthase comprise a contiguous DNA molecule.

## Patentansprüche

1. Verfahren zur biokatalytischen Verbesserung der Herstellung von einer aromatischen Verbindung in einem E. coli Zelltransformanden über den gemeinsamen Syntheseweg der Biosynthese von aromatischen Aminosäuren, die zu der Zelle endogen ist, wobei das Verfahren den Schritt aufweist:
Kultivierung des Zelltransformanden in Medien enthaltend eine metabolisierbare Kohlenstoffquelle unter Bedingungen, unter denen die Metabolisierung der Kohlenstoffquelle durchführbar ist, wobei der Zelltransformand exogene DNA-Frequenzen aufweist, die Enzymarten des gemeinsamen Syntheseweges kodieren, wobei die Enzymarten im wesentlichen aus den Enzymen Transketolase, 3-Desoxy-D-arabinoheptulosonat-7-phosphatsynthase, 3-Dehydrochinatsynthase, Shikimatkinase, 5-Enolpyruvoylshikimat-3-phosphatsynthase und Chorismatsynthase bestehen.

2. Verfahren nach Anspruch 1, **dadurch gekennzeichnet, dass** ein oder mehrere rekombinante Plasmidvektoren die exogenen DNA-Sequenzen, die die Enzymart kodieren, aufweisen.

3. E. coli-Transformand, **gekennzeichnet durch** die verstärkte Expression von strukturellen Genen, die die Enzymarten Transketolase, 3-Desoxy-D-arabino-heptulosonat-7-phosphatsynthase, 3-Dehydrochinat-synthase, Shikimatkinase, 5-Enolpyruvoylshikimat-3-phosphatsynthase und Chorismatsynthase kodieren.

4. Verfahren zur biokatalytischen Verbesserung der Herstellung einer aromatischen Verbindung aus einer Kohlenstoffquelle, wobei das Verfahren den Schritt der Kultivierung des Zelltransformanden nach Anspruch 3 in Medien enthaltend eine metabolisierbare Kohlenstoffquelle unter Bedingungen, bei denen die Metabolisierung der Kohlenstoffquelle durchführbar ist, aufweist.

5. Plasmidkonstrukt mit strukturellen Genen für Enzymarten des gemeinsamen Syntheseweges, wobei die Enzymarten im wesentlichen aus 3-Dehydrochinatsynthase, Shikimatkinase, 5-Enolpyruvoyl-shikimat-3-phosphatsynthase und Chorismatsynthase bestehen.

6. Prokaryontischer Zelltransformand ausgewählt aus der Gruppe von Prokaryonten, die zu den Gattungen Escherichia, Corynebakterium, Brevibakteria, Arthrobacter, Bazillus, Pseudomonas, Streptomyces, Staphlococcus oder Seratia gehören, **gekennzeichnet durch** die Expression von exogenen strukturellen Genen, die die Enzymarten Transketolase, 3-Desoxy-D-arabino-heptulosonat-7-phosphatsynthase, 3-Dehydrochinatsynthase, Shikimatkinase, 5-Enolpyruvoyl-shikimat-3-phosphatsynthase und Chorismatsynthase kodieren.

7. Plasmid pKAD50, wie in Figur 10 gezeigt.

8. Verfahren nach Anspruch 1, **dadurch gekennzeichnet, dass** die exogenen DNA-Sequenzen für Shikimatkinase, 5-Enolpyruvoylshikimat-3-phosphatsynthase und Chorismatsynthase ein angrenzendes DNA-Molekül aufweisen.

## Revendications

1. Procédé pour augmenter la production biocatalytique d'un composé aromatique dans une cellule transformée d'E. coli par la voie commune de la biosynthèse d'aminoacides aromatiques endogène à ladite cellule, ce procédé comprenant l'étape de culture de la cellule transformée dans des milieux contenant une source de carbone métabolisable dans des conditions qui entraînent la métabolisation de ladite source de carbone, ladite cellule transformée comprenant des séquences d'ADN exogènes codant pour des espèces enzymatiques de la voie commune, lesdites espèces enzymatiques étant essentiellement constituées des enzymes transkétolase, 3-désoxy-D-arabino-heptulosonate-7-phosphate-synthase, 3-déshydroquinate-synthase, shikimate-kinase, 5-énolpyruvoylshikimate-3-phosphatesynthase et chorismate-synthase.

2. Procédé selon la revendication 1, dans lequel un ou plusieurs vecteurs plasmidiques recombinés comprennent les séquences d'ADN exogènes codant pour lesdites espèces enzymatiques.

3. Cellule transformée d'E. coli **caractérisée par** une expression amplifiée de gènes de structure codant pour les espèces enzymatiques transkétolase, 3-désoxy-D-arabino-heptulosonate-7-phosphate-synthase, 3-déshydroquinate-synthase, shikimate-kinase, 5-énolpyruvoylshikimate-3-phosphate-synthase et chorismate-synthase.

4. Procédé pour augmenter la production biocatalytique d'un composé aromatique à partir d'une source de carbone, ledit procédé comprenant l'étape de culture de la cellule transformée de la revendication 3 dans des milieux contenant une source de carbone métabolisable dans des conditions entraînant la métabolisation de ladite source de carbone.

5. Assemblage plasmidique comprenant des gènes de structure pour des espèces enzymatiques d'une voie commune, lesdites espèces enzymatiques étant constituées essentiellement de la 3-déshydroquinate-synthase, de la shikimate-kinase, de la 5-énolpyruvoylshikimate-3-phosphate-synthase et de la chorismate-synthase.

6. Cellule procaryote transformée choisie dans le groupe de procaryotes appartenant aux genres *Escherichia, Brevibacterium, Arthrobacter, Bacillus, Pseudomonas, Streptomyces, Staphylococcus* ou *Serratia*, et **caractérisée par** l'expression de gènes de structure exogènes codant pour les espèces enzymatiques transkétolase, 3-désoxy-D-arabino-heptulosonate-7-phosphate-synthase, 3-déshydroquinate-synthase, shikimate-kinase, 5-énolpyruvoylshikimate-3-phosphatesynthase et chorismate-synthase.

7. Plasmide pKAD50, tel que représenté sur la figure 10.

8. Procédé selon la revendication 1, dans lequel les séquences d'ADN codant pour la shikimate-kinase, la 5-énolpyruvoylshikimate-3-phosphate-synthase et la chorismate-synthase comprennent une molécule d'ADN contiguë.
